# FASCICULE DE BREVET EUROPEEN

(11) **EP 1 075 825 B1**
(45) Date de publication et mention de la délivrance du brevet: **04.08.2004**
(21) Numéro de dépôt: 00400709.2
(22) Date de dépôt: 15.03.2000
(51) Int. Cl.: A61F 2/06

(54) **Prothèse aortique bifurquée**
Verzweigte intraaortale Prothese
Bifurcated aortic prosthesis

(30) Priorité: 09.08.1999 FR 9910319
(43) Date de publication de la demande: 14.02.2001
(73) Titulaire: Novatech SA, 06130 Plan de Grasse (FR)
(72) Inventeur: Fedida, José, 06370 Mouans-Sartoux (FR)
(74) Mandataire: Hauer, Bernard

(56) Documents cités:
- WO-A-98/06355
- WO-A-99/43273
- FR-A- 2 722 678
- US-A- 5 683 449
- US-A- 5 693 088

## Description

La présente invention concerne une prothèse aortique bifurquée.

Cette prothèse est destinée à être implantée dans l'aorte abdominale d'un corps humain pour traiter un anévrisme de la bifurcation aortique, c'est-à-dire un anévrisme qui est situé sur l'aorte abdominale entre l'embranchement des artères rénales et la bifurcation des artères iliaques, généralement à proximité de cette bifurcation.

De nombreux exemples de telles prothèses sont connus. On citera, à titre d'illustration, les documents WO-97/40779, WO-95/21592, EP-0 880 948 et EP-0 461 791.

Le document WO-95/21592 divulgue notamment une prothèse aortique bifurquée comportant :
- une structure qui comprend un tronc principal se divisant en deux jambes de même longueur et qui est réalisée de manière à permettre à ladite prothèse de prendre l'une de deux positions : une position repliée et compacte pour permettre l'implantation de la prothèse dans ladite aorte et une position implantée et déployée dans l'aorte pour créer un canal de substitution au niveau dudit anévrisme ; et
- au moins une enveloppe étanche enveloppant, au moins partiellement, ladite structure.

Cette prothèse présente une taille réduite et elle est agencée à proximité des artères rénales de sorte qu'elle ne couvre qu'une partie réduite de l'anévrisme. Deux jambages qui pénètrent jusque dans les artères iliaques sont liés respectivement auxdites jambes de la prothèse. Ces jambages couvrent ainsi le reste de l'anévrisme.

Ce mode de réalisation présente l'inconvénient de faire remonter, au niveau des artères rénales, le tourbillon sanguin qui est provoqué par la bifurcation aortique (c'est-à-dire la bifurcation des artères iliaques) et qui est généralement responsable de l'anévrisme, ce qui est bien entendu néfaste à moyen terme et est susceptible de provoquer un nouvel anévrisme, à proximité desdites artères rénales.

De plus, la structure de cette prothèse est telle qu'elle nécessite la génération d'une force importante pour amener ladite prothèse, lors de son implantation, de la position repliée dans la position déployée. Ce document connu enseigne d'utiliser un ballon gonflable pour engendrer une telle force.

Toutefois, une telle utilisation est difficile à mettre en oeuvre et elle est de plus difficilement contrôlable. En outre, le gonflement du ballon bloque complètement la circulation sanguine, ce qui rend nécessaire de prévoir des moyens ou des procédures spécifiques pour remédier aux effets de l'interruption provisoire de la circulation sanguine.

Le document EP-0 461 791 propose, comme alternative à cette mise en oeuvre, une prothèse qui comprend une structure à mémoire de forme, ce qui permet à la structure de revenir automatiquement dans la configuration initiale, notamment la configuration déployée, sans utiliser de moyens spécifiques à cet effet.

La prothèse décrite dans ce document connu se différencie de plus de celle divulguée par le document WO-95/21592 en ce qu'elle présente une taille globale beaucoup plus importante, c'est-à-dire à la fois du tronc et des jambes, de telle sorte que les jambes pénètrent dans les artères iliaques, et ceci sur une distance relativement importante. De plus, dans un mode de réalisation particulier, l'enveloppe recouvre complètement lesdites jambes et partiellement ledit tronc. L'enveloppe qui pénètre donc largement dans les artères iliaques, se termine à l'autre extrémité bien en deçà des artères rénales.

Cette prothèse connue présente comme inconvénient majeur une mise en place qui est particulièrement difficile et qui doit être réalisée selon une procédure longue et complexe. En effet, dans ce cas, la prothèse est tout d'abord amenée à partir d'une artère fémorale, via une artère iliaque, dans l'aorte au niveau de la bifurcation aortique et donc de l'anévrisme. A cet effet, les deux jambes de la prothèse sont jointes et introduites dans un même dispositif introducteur. Après retrait de ce dernier, les deux jambes sont situées dans une seule et même artère iliaque. Il est alors nécessaire d'extraire l'une desdites jambes de cette artère iliaque et de l'amener dans l'autre artère iliaque.

La mise en place de cette prothèse est donc particulièrement délicate et longue.

La présente invention a pour objet de remédier à ces inconvénients. Elle concerne une prothèse aortique bifurquée destinée à être implantée dans l'aorte d'un corps humain pour traiter un anévrisme de la bifurcation aortique, prothèse qui peut être implantée de façon simple et rapide, qui permet de traiter efficacement l'anévrisme et qui évite de plus une remontée du tourbillon sanguin précité au niveau des artères rénales.

A cet effet, ladite prothèse, du type connu et décrit notamment dans le document EP-0 461 791, comportant :
- une structure qui comprend un tronc principal se divisant en deux jambes de même longueur, qui est du type à mémoire de forme et qui est réalisée de manière à permettre à ladite prothèse de prendre l'une de deux positions : une position repliée et compacte pour permettre l'implantation de la prothèse dans ladite aorte et une position implantée et déployée dans l'aorte pour créer un canal de substitution au niveau dudit anévrisme ; et
- au moins une enveloppe étanche enveloppant complètement lesdites jambes et au moins partiellement ledit tronc,
est remarquable, selon l'invention, en ce que ladite enveloppe présente une longueur telle que, dans ladite position implantée, elle couvre sensiblement la partie de l'aorte comprise entre les artères rénales et les artères iliaques, et en ce que la longueur desdites jambes est comprise entre 1/4 et 2/5 et est proche, de préférence, de 1/3 de la longueur de ladite enveloppe.

Ainsi, grâce à l'invention :
- comme les jambes de la structure qui sont complètement recouvertes par l'enveloppe ne pénètrent pas dans les artères iliaques, la mise en place de la prothèse est simplifiée et facilitée, notamment par rapport à la solution précitée préconisée par le document EP-0 461 791. De plus, la longueur desdites jambes, bien que réduite, est suffisante pour permettre une prise aisée et donc pour permettre en particulier un assemblage facile de jambages additionnels aux extrémités libres desdites jambes. La mise en place de la prothèse est bien entendu facilitée par l'utilisation d'une structure à mémoire de forme ;
- comme toute la zone entre les artères iliaques et les artères rénales est couverte par l'enveloppe, tout l'anévrisme est traité (par le canal de substitution créé par la prothèse). Bien entendu, ladite enveloppe est réalisée en un matériau biocompatible qui, tout en étant étanche au flux sanguin, reste perméable aux échanges cellulaires ; et
- comme la partie de tronc recouverte par l'enveloppe présente une longueur importante (comprise entre 3/5 et 3/4 de la distance existant entre les aortes iliaques et les artères rénales), le tourbillon sanguin provoqué par la bifurcation aortique s'atténue au moins fortement, sinon complètement, dans cette partie couverte et ne remonte donc pas au niveau des artères rénales.

Selon l'invention, le diamètre desdites jambes est sensiblement égal à la moitié du diamètre dudit tronc principal. Par conséquent, le diamètre des jambes est plus important que dans les prothèses connues précitées, ce qui permet d'améliorer la circulation sanguine au niveau du raccordement des jambes au tronc de la prothèse.

De façon avantageuse, au moins l'une desdites jambes est évasée à son extrémité libre, ce qui facilite l'assemblage d'un jambage additionnel qui est implanté dans l'une des artères iliaques.

Par ailleurs, avantageusement, ladite enveloppe présente, au niveau de l'extrémité libre d'au moins l'une desdites jambes, un retour, ce qui permet d'assurer une étanchéité avec le jambage additionnel qui est assemblé à ladite jambe.

De plus, pour obtenir une fixation solide et durable de l'enveloppe sur la structure, ladite enveloppe est cousue au moins à son extrémité qui est opposée à l'extrémité libre des jambes.

Par ailleurs, avantageusement, la prothèse conforme à l'invention comporte au moins un élément radio-opaque, ce qui permet de détecter facilement et de situer exactement, par radiographie, dans le corps humain la prothèse, lors de son implantation et/ou après son implantation. Le ou les éléments radio-opaques utilisés, par exemple des éléments connus et rapportés de façon spécifique ou de préférence des moyens de liaison décrits ci-dessous, peuvent être prévus à différents endroits de la structure. Avantageusement, la prothèse conforme à la présente invention comporte :
- au moins un élément radio-opaque agencé à l'extrémité libre d'au moins l'une desdites jambes ; et/ou
- au moins deux éléments radio-opaques agencés sur ladite structure de manière à délimiter, dans la position implantée, l'embranchement d'au moins l'une des artères rénales ; et/ou
- une pluralité d'éléments radio-opaques agencés en forme de V et reliant le point de bifurcation entre les deux jambes, respectivement aux deux côtés du tronc, à l'extrémité de l'enveloppe opposée aux jambes.

Dans un mode de réalisation préféré, ladite structure est réalisée au moins partiellement sous forme d'un treillis et comporte au moins un fil ondulé formant des unités sensiblement annulaires liées entre elles. Au moins certaines des ondulations dudit fil ondulé, respectivement de deux unités adjacentes, sont liées deux à deux par des moyens de liaison. Au moins certains desdits moyens de liaison comportent des maillons qui sont réalisés en une pièce rigide et qui sont munis au moins de deux boucles solidaires l'une de l'autre, et, pour chacun desdits maillons, chacune des deux boucles dudit maillon emprisonne, avec du jeu, respectivement les deux ondulations à lier.

Dans le cadre de la présente invention, certains desdits maillons peuvent être radio-opaques et être utilisés comme éléments radio-opaques pour permettre la détection de la prothèse.

Les figures du dessin annexé feront bien comprendre comment l'invention peut être réalisée. Sur ces figures, des références identiques désignent des éléments semblables.

La figure 1 illustre schématiquement une prothèse aortique bifurquée conforme à l'invention.

Les figures 2 et 3 montrent schématiquement des jambages, respectivement dans deux modes de réalisation différents, jambages qui sont susceptibles d'être rendus solidaires de jambes de la prothèse de la figure 1.

Les figures 4 et 5 illustrent schématiquement des moyens de liaison préférés pour la structure de la prothèse représentée sur la figure 1, respectivement dans des positions fermée et ouverte.

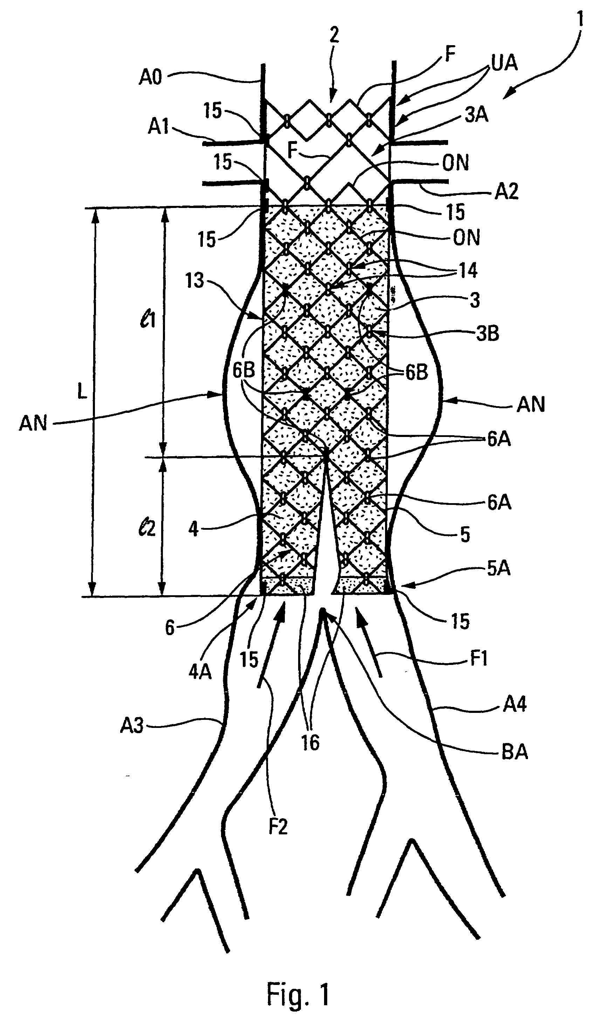

La prothèse aortique bifurquée 1 conforme à l'invention et représentée schématiquement sur la figure 1 est destinée à être implantée dans l'aorte abdominale AO d'un corps humain pour traiter un anévrisme de la bifurcation aortique BA, c'est-à-dire un anévrisme AN qui est situé sur l'aorte abdominale AO entre l'embranchement des artères rénales A1 et A2 et la bifurcation BA des artères iliaques A3 et A4.

De façon connue, ladite prothèse 1 comporte :
- une structure 2 qui comprend un tronc principal 3 qui se divise en deux jambes 4, 5 et qui est réalisée de manière à permettre à ladite prothèse de prendre l'une des deux positions suivantes : une position repliée et compacte, pour permettre l'implantation de la prothèse 1 dans ladite aorte abdominale AO, et une position implantée et déployée dans l'aorte AO, comme représenté sur la figure 1, pour créer un canal de substitution au niveau dudit anévrisme AN ; et
- une enveloppe étanche 6, par exemple en textile polyester, qui recouvre en partie ladite structure 2. Bien entendu, ladite enveloppe 6 doit être réalisée en un matériau biocompatible qui, tout en étant étanche au flux sanguin, reste perméable aux échanges cellulaires.

En particulier, pour faciliter l'implantation de la prothèse 1, la structure 2 est une structure à mémoire de forme, ce qui permet à la prothèse 1 qui est initialement dans la position déployée et qui est contrainte dans la position repliée en vue de son implantation, de revenir automatiquement dans ladite position déployée après son introduction dans l'aorte AO, comme représenté sur la figure 1. Aucun moyen spécifique, tel qu'un ballon gonflable, n'est donc nécessaire pour amener la prothèse 1 dans la position (déployée) de fonctionnement.

Selon l'invention, comme on peut le voir sur la figure 1 :
- l'enveloppe étanche 6 enveloppe complètement les deux jambes 4 et 5 et partiellement le tronc 3 (sur une longueur ℓ1) de telle sorte qu'elle couvre sensiblement la partie de l'aorte AO comprise entre les artères rénales A1 et A2 et les artères iliaques A3, A4 ; et
- la longueur ℓ2 desdites jambes 4 et 5 est comprise entre 1/4 et 2/5, et est proche de préférence de 1/3, de la longueur L de ladite enveloppe 6.

Généralement, bien que non exclusivement :
- la longueur ℓ1 est de l'ordre de 7 à 8 cm ; et
- la longueur ℓ2 est de l'ordre de 4 à 5 cm.

Ainsi, grâce à l'invention :
- comme les jambes 4, 5 (de même longueur) de la structure 2 qui sont complètement recouvertes par l'enveloppe 6 ne pénètrent pas dans les artères iliaques A3, A4, la mise en place de la prothèse 1 est simplifiée et facilitée. Cette mise en place est bien entendu facilitée par l'utilisation d'une structure à mémoire de forme ;
- comme toute la zone entre les artères iliaques A3, A4 et les artères rénales A1, A2 est couverte, tout l'anévrisme AN est traité (par le canal de substitution créé par la prothèse 1); et
- comme la partie 3B du tronc 3, recouverte par l'enveloppe 6, présente une longueur ℓ1 importante (comprise entre 3/5 et 3/4 de la distance, sensiblement égale à L, existant entre les artères iliaques A3, A4 et les artères rénales A1, A2), le tourbillon sanguin provoqué par la bifurcation aortique est fortement, voire complètement, amorti dans cette partie 3B couverte et ne remonte donc pas au niveau des artères rénales A1, A2.

On notera que, selon l'invention, les jambes 4 et 5 présentent de plus un diamètre "important", par exemple de l'ordre de 12 à 14 mm, qui est de préférence sensiblement égal à la moitié du diamètre du tronc 3, ce qui favorise une bonne circulation sanguine entre ledit tronc 3 et lesdites jambes 4 et 5.

De plus, la longueur ℓ2 des jambes 4 et 5 qui est une longueur "moyenne" permet une mise en place aisée de jambages 7, 8 aux extrémités libres 4A, 5A de ces dernières, comme illustré sur la figure 1 par des flèches F2 et F1 qui représentent le sens de mise en place de tels jambages 7, 8.

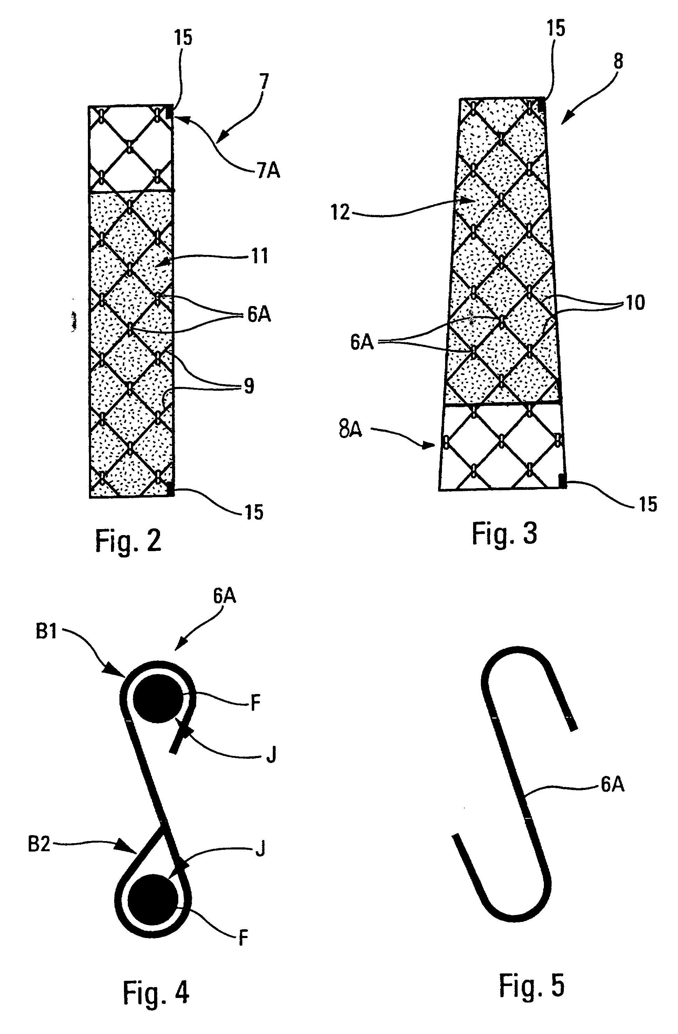

Ces jambages 7, 8 comportent chacun, comme représenté sur les figures 2 et 3 :
- une structure 9, 10, de préférence de même type que la structure 2 de la prothèse 1, qui présente par exemple une forme cylindrique, comme la structure 9 représentée sur la figure 2 ou une forme tronconique, comme la structure 10 représentée sur la figure 3 ; et
- une enveloppe étanche 11, 12, de préférence de même type que l'enveloppe 6 de la prothèse 1, qui recouvre ladite structure 9, 10, à l'exception d'une petite partie, par exemple de 1 centimètre, à l'extrémité 7A, 8A qui est destinée à être assemblée à l'une desdites jambes 4, 5 de la prothèse 1. Comme on peut le voir, les dispositions des jambages 7 et 8 sont inversées entre les figures 2 et 3.

Pour faciliter cet assemblage, la jambe 5 est évasée à son extrémité libre 5A.

On notera qu'une telle caractéristique (extrémité évasée) n'est pas obligatoirement nécessaire pour la mise en place de l'une desdites jambes de la prothèse 1, en l'occurrence la jambe 4, puisque l'on utilise généralement pour celle-ci un guide connu et utilisé préalablement pour la mise en place de la prothèse 1 de sorte que le guidage du jambage associé est généralement très précis et suffisant. Toutefois, la jambe 4 peut, bien entendu, également être évasée à son extrémité libre 4A.

En outre, selon l'invention, l'enveloppe 6 présente un retour 16 au niveau des jambes 4, 5, ce qui permet notamment d'assurer une bonne étanchéité de l'ensemble formé par la prothèse 1 et les jambages, après la mise en place de ces derniers.

De plus, pour obtenir une fixation solide de l'enveloppe 6 sur la structure 2, ladite enveloppe 6 est cousue au moins à ses extrémités.

Comme indiqué précédemment, le tronc 3 de la structure 2 présente une partie 3B couverte par l'enveloppe 6 et une partie 3A non couverte. Selon l'invention, ladite partie 3A qui remonte au-delà des artères rénales A1, A2 permet de fixer facilement la prothèse 1 sur une partie saine de l'aorte A0 au niveau desdites artères rénales A1 et A2.

De plus, comme on peut le voir sur la figure 1, ladite structure 2 présente, au niveau de l'embranchement des artères rénales A1 et A2, un maillage élargi de manière à ne pas gêner ou perturber la circulation sanguine entre l'aorte AO et lesdites artères rénales A1 et A2.

Dans le cadre de la présente invention, la structure 2 de la prothèse 1 peut présenter différentes formes connues.

De préférence, toutefois, ladite structure 2 comprend, de façon connue, au moins un treillis 13 qui est au moins en partie cylindrique et qui comporte au moins un fil ondulé F formant des unités ondulés UA sensiblement annulaires, liées entre elles. Ces unités annulaires ondulées UA comportent une pluralité d'ondulations ON. De plus, au moins certaines de ces ondulations ON, à chaque fois de deux unités UA adjacentes, sont liées deux à deux par des moyens de liaison 14, de manière à relier ensemble lesdites unités annulaires UA adjacentes et à ainsi former ledit treillis cylindrique 13.

Dans le cadre de la présente invention, chaque unité annulaire UA peut être réalisée au moyen d'un fil F spécifique de sorte que les différentes unités annulaires UA sont alors, avant liaison, complètement indépendantes les unes des autres.

Toutefois, il est également possible de réaliser l'ensemble des unités annulaires UA avec un seul et même fil F. A cet effet, le fil F est configuré de manière à passer, après création d'une unité annulaire UA, à l'unité annulaire suivante UA.

Selon l'invention, notamment pour permettre une liaison solide, souple, durable et non blessante pour l'aorte AO, au moins certains des moyens de liaison 14 comportent des maillons 6A, 6B spécifiques décrits ci-dessous.

Selon l'invention, un tel maillon 6A, 6B est réalisé dans une pièce rigide et comporte, comme représenté sur les figures 4 et 5 pour le maillon 6A, au moins deux boucles B1 et B2, dont chacune emprisonne, avec un jeu J, l'une des ondulations ou parties de fil F à lier ensemble. Les boucles B1 et B2 sont telles que les parties de fil (emprisonnées avec jeu), tout en étant rendues solidaires, peuvent se mouvoir (tourner) librement, ce qui permet d'obtenir une liaison présentant les caractéristiques précitées.

En effet :
- la liaison est solide et durable, grâce à la rigidité du maillon 6A et à la génération de boucles B1 et B2 dans la pièce même du maillon 6A ;
- la liaison est très souple, en particulier grâce au jeu J et à la séparation des deux parties de fil F devant être liées l'une à l'autre ; et
- la liaison ne comprend, par exemple, aucune partie saillante et il n'existe donc aucun danger de blesser la paroi de l'aorte AO, dans laquelle est implantée la prothèse 1.

On notera que la figure 4 illustre une position fermée ou position de liaison du maillon 6A et la figure 5 illustre la position ouverte de ce maillon 6A avant la liaison.

Dans le cadre de la présente invention :
- le fil F et les maillons 6A peuvent être réalisés en métal ou par exemple en un matériau connu commercialisé sous le nom de "nitinol" ;
- les boucles, comme la boucle B2 de la figure 4, peuvent être complètement fermées, ce qui assure alors une liaison extrêmement solide ;
- lesdites boucles peuvent également n'être que partiellement fermées, c'est-à-dire juste suffisamment pour emprisonner efficacement la partie de fil ou ondulation à lier, comme cela est le cas pour la boucle B1 de la figure 4, ce qui facilite bien entendu la réalisation des boucles.

On notera que la partie 3A du tronc 3 de ladite structure 2 comporte, par exemple, une seule unité annulaire UA en aval des artères rénales A1, A2 et deux unités annulaires UA en amont de celles-ci. De plus, l'unité annulaire située directement au niveau desdites artères rénales A1, A2 présente des ondulations qui sont étendues de manière à ne pas perturber ou bloquer la circulation sanguine entre l'aorte AO et ces artères rénales A1, A2.

Par ailleurs, pour permettre de détecter facilement et de situer exactement la prothèse 1, lors de son implantation dans l'aorte AO ou après cette implantation, ladite prothèse 1 comporte des éléments radio-opaques 6B, 15. Il en est de même pour les jambages 7 et 8, comme représenté sur les figures 2 et 3.

Concernant ces éléments radio-opaques, il peut s'agir :
- soit d'éléments radio-opaques 15 usuels, rapportés de façon spécifique ;
- sont des maillons 6B qui sont de même forme que les maillons 6A décrits précédemment, ces maillons 6B étant de plus rendus radio-opaques par l'utilisation d'un matériau ou d'un revêtement approprié.

Comme on peut le voir sur la figure 1, la prothèse 1 comporte :
- un élément radio-opaque 15 à l'extrémité de chacune des jambes 4 et 5 ;
- deux éléments radio-opaques 15 délimitant, dans la position implantée, l'une A1 des artères rénales ; et
- une pluralité d'éléments radio-opaques 6B, 15 agencés en forme de V et reliant le point de bifurcation entre les deux jambes 4 et 5, respectivement aux deux côtés du tronc 3, à l'extrémité de l'enveloppe 6 opposée aux jambes 4 et 5.

## Revendications

1. Prothèse aortique bifurquée destinée à être implantée dans l'aorte abdominale (AO) d'un corps humain pour traiter un anévrisme (AN) de la bifurcation aortique (BA), ladite prothèse (1) comportant :
- une structure (2) qui comprend un tronc principal (3) se divisant en deux jambes (4, 5) de même longueur, qui est du type à mémoire de forme et qui est réalisée de manière à permettre à ladite prothèse (1) de prendre l'une de deux positions : une position repliée et compacte pour permettre l'implantation de la prothèse (1) dans ladite aorte (AO) et une position implantée et déployée dans l'aorte (AO) pour créer un canal de substitution au niveau dudit anévrisme (AN) ; et
- au moins une enveloppe étanche (6) enveloppant complètement lesdites jambes (4, 5) et au moins partiellement ledit tronc (3),
**caractérisée en ce que** ladite enveloppe (6) présente une longueur (L) telle que, la longueur (ℓ2) desdites jambes (4, 5) est comprise entre 1/4 et 2/5 de la longueur (L) de ladite enveloppe (6), et la longueur (ℓ1) de la partie (3B) dudit tronc (3), recouverte par ladite enveloppe (6), est comprise entre 3/5 et 3/4 de la distance existant entre les artères rénales (A1, A2) et les artères iliaques (A3, A4) ladite enveloppe (6) couvrant sensiblement la partie de l'aorte (AO) comprise entre les artères rénales (A1, A2) et les artères iliaques (A3, A4), dans ladite position implantée.

2. Prothèse selon la revendication 1,
**caractérisée en ce que** la longueur (ℓ2) des jambes (4, 5) représente sensiblement 1/3 de la longueur (L) de l'enveloppe (6).

3. Prothèse selon l'une des revendications 1 et 2,
**caractérisée en ce que** le diamètre desdites jambes (4, 5) est sensiblement égal à la moitié du diamètre dudit tronc principal (3).

4. Prothèse selon l'une des revendications 1 à 3,
**caractérisée en ce qu'**au moins l'une (5) desdites jambes (4, 5) est évasée à son extrémité libre (5A).

5. Prothèse selon l'une des revendications 1 à 4,
**caractérisée en ce que** ladite enveloppe (6) présente, au niveau de l'extrémité libre (4A, 5A) d'au moins l'une desdites jambes (4, 5), un retour (16).

6. Prothèse selon l'une quelconque des revendications précédentes,
**caractérisée en ce que** ladite enveloppe (6) est cousue au moins à son extrémité qui est opposée à l'extrémité libre (4A, 5A) des jambes (4, 5).

7. Prothèse selon l'une quelconque des revendications précédentes,
**caractérisée en ce qu'**elle comporte au moins un élément radio-opaque (6B, 15).

8. Prothèse selon la revendication 7,
**caractérisée en ce qu'**elle comporte au moins un élément radio-opaque (15) agencé à l'extrémité libre (4A, 5A) d'au moins l'une desdites jambes (4, 5).

9. Prothèse selon l'une des revendications 7 et 8,
**caractérisée en ce qu'**elle comporte au moins deux éléments radio-opaques (15) agencés sur ladite structure (2) de manière à délimiter, dans la position implantée, l'embranchement d'au moins l'une (A1) des artères rénales (A1, A2).

10. Prothèse selon l'une des revendications 7 à 9,
**caractérisée en ce qu'**elle comporte une pluralité d'éléments radio-opaques (6B, 15) agencés en forme de V et reliant le point de bifurcation entre les deux jambes (4, 5), respectivement aux deux côtés du tronc (3), à l'extrémité de l'enveloppe (6) opposée aux jambes (4, 5).

11. Prothèse selon l'une quelconque des revendications précédentes,
**caractérisée en ce que** ladite structure (2) présente une partie d'extrémité (3A) non recouverte par l'enveloppe (6) et destinée à la fixation de la structure (2) sur l'aorte (AO) au niveau des artères rénales (A1, A2).

12. Prothèse selon l'une quelconque des revendications précédentes,
**caractérisée en ce que** ladite structure (2) est réalisée au moins partiellement sous forme d'un treillis (13) et comporte au moins un fil ondulé (F) formant des unités (UA) sensiblement annulaires liées entre elles, **en ce qu'**au moins certaines des ondulations (ON) dudit fil ondulé (F), respectivement de deux unités adjacentes (UA), sont liées deux à deux par des moyens de liaison (14), **en ce qu'**au moins certains desdits moyens de liaison (14) comportent des maillons (6A, 6B) qui sont réalisés en une pièce rigide et qui sont munis au moins de deux boucles (B1, B2) solidaires l'une de l'autre, et **en ce que**, pour chacun desdits maillons (6A, 6B), chacune des deux boucles (B1, B2) dudit maillon emprisonne, avec du jeu (J), respectivement les deux ondulations (ON) à lier.

13. Prothèse selon la revendication 12,
**caractérisée en ce qu'**au moins l'un (6B) desdits maillons (6A, 6B) est radio-opaque.

## Claims

1. Bifurcated aortic prosthesis intended to be implanted in the abdominal aorta (AO) of a human body to treat an aneurysm (AN) of the aortic bifurcation (BA), said prosthesis (1) comprising:
- a structure (2) which comprises a main trunk (3) which divides into two branches (4, 5) of equal lengths, which is of the shape memory type and which is produced in such a way as to allow said prosthesis (1) to adopt one of two positions: a folded and compact position to allow the prosthesis (1) to be implanted in said aorta (AO) and a position in which it is implanted and deployed in the aorta (AO) to create a substitute passage in the region of said aneurysm (AN); and
- at least one impervious envelope (6) completely enveloping said branches (4, 5) and at least partially enveloping said trunk (3),
**characterized in that** said envelope (6) has a length (L) such that, the length (12) of said branches (4, 5) is between 1/4 and 2/5 of the length (L) of said envelope (6), and the length (11) of the part (3B) of said trunk (3), covered by said envelope (6), is between 3/5 and 3/4 of said distance between the renal arteries (A1, A2) and the iliac arteries (A3, A4), said envelope (6) covering roughly that part of the aorta (AO) which lies between the renal arteries (A1, A2) and the iliac arteries (A3, A4) in said implanted position.

2. Prosthesis according to Claim 1, **characterized in that** the length (12) of the branches (4, 5) represents approximately 1/3 of the length (L) of the envelope (6).

3. Prosthesis according to one of Claims 1 and 2, **characterized in that** the diameter of said branches (4, 5) is approximately equal to half the diameter of said main trunk (3).

4. Prosthesis according to one of Claims 1 to 3, **characterized in that** at least one (5) of said branches (4, 5) is flared at its free end (5A).

5. Prosthesis according to one of Claims 1 to 4, **characterized in that**, at the free end (4A, 5A) of at least one of said branches (4, 5) said envelope (6) has a turned-back region (16).

6. Prosthesis according to any one of the preceding claims, **characterized in that** said envelope (6) is sewn at least at its opposite end to the free end (4A, 5A) of the branches (4, 5).

7. Prosthesis according to any one of the preceding claims, **characterized in that** it comprises at least one radio-opaque element (6B, 15).

8. Prosthesis according to Claim 7, **characterized in that** it comprises at least one radio-opaque element (15) arranged at the free end (4A, 5A) of at least one of said branches (4, 5).

9. Prosthesis according to one of Claims 7 and 8, **characterized in that** it comprises at least two radio-opaque elements (15) arranged on said structure (2) in such a way as, in the implanted position, to delimit the branch connection of at least one (A1) of the renal arteries (A1, A2).

10. Prosthesis according to one of Claims 7 to 9, **characterized in that** it comprises a number of radio-opaque elements (6B, 15) arranged in a V-shape and connecting the fork between the two branches (4, 5), on the two sides of the trunk (3) respectively, to the opposite end of the envelope (6) to the branches (4, 5).

11. Prosthesis according to any one of the preceding claims, **characterized in that** said structure (2) has an end part (3A) which is not covered by the envelope (6) and which is intended for fixing the structure (2) to the aorta (AO) in the region of the renal arteries (A1, A2).

12. Prosthesis according to any one of the preceding claims, **characterized in that** said structure (2) is at least partially produced in the form of a mesh (13) and has at least one corrugated filament (F) forming approximately annular units (UA) linked together; at least some of the corrugations (ON) of said corrugated filament (F) of two adjacent units (UA) respectively are linked to one another by linking means (14); at least some of said linking means (14) comprise links (6A, 6B) which are made as a rigid piece and provided with at least two loops (B1, B2) joined together; and, in the case of each of said links (6A, 6B), each of the two loops (B1, B2) of said link entraps, with some clearance (J), a respective one of the two corrugations (ON) that are to be linked.

13. Prosthesis according to Claim 12, **characterized in that** at least one (6B) of said links (6A, 6B) is radio-opaque.

## Patentansprüche

1. Verzweigte aortale Prothese, die dazu bestimmt ist, in die Bauchaorta (AO) eines menschlichen Körpers implantiert zu werden, um ein Aneurysma (AN) der Aortengabelung (BA) zu behandeln, wobei die Prothese (1) umfasst:
- eine Struktur (2), die einen Hauptstamm (3) umfasst, der sich in zwei Schenkel (4, 5) von gleicher Länge teilt, die vom Typ mit Formgedächtnis ist und die so ausgebildet ist, dass sie der Prothese (1) ermöglicht, eine von zwei Positionen einzunehmen: eine zusammengefaltete und kompakte Position, um die Implantation der Prothese (1) in die Aorta (AO) zu ermöglichen, und eine implantierte und in der Aorta (AO) entfaltete Position, um im Bereich des Aneurysma (AN) einen Ersatzkanal zu schaffen; und
- mindestens eine dichte Umhüllung (6), welche die Schenkel (4, 5) vollständig und den Stamm (3) mindestens teilweise umhüllt,
**dadurch gekennzeichnet, dass** die Umhüllung (6) eine solche Länge (L) aufweist, dass die Länge (ℓ2) der Schenkel (4, 5) zwischen 1/4 und 2/5 der Länge (L) der Umhüllung (6) beträgt und die Länge (ℓ1) des Teils (3B) des Stamms (3), der von der. Umhüllung (6) bedeckt ist, zwischen 3/5 und 3/4 der Entfernung beträgt, die zwischen den Nierenarterien (A1, A2) und den Hüftarterien (A3, A4) besteht, wobei die Umhüllung (6) in der implantierten Position im Wesentlichen den Teil der Aorta (AO) zwischen den Nierenarterien (A1, A2) und den Hüftarterien (A3, A4) bedeckt.

2. Prothese nach Anspruch 1, **dadurch gekennzeichnet, dass** die Länge (ℓ2) der Schenkel (4, 5) im Wesentlichen 1/3 der Länge (L) der Umhüllung (6) beträgt.

3. Prothese nach einem der Ansprüche 1 und 2, **dadurch gekennzeichnet, dass** der Durchmesser der Schenkel (4, 5) im Wesentlichen gleich der Hälfte des Durchmessers des Hauptstamms (3) ist.

4. Prothese nach einem der Ansprüche 1 bis 3, **dadurch gekennzeichnet, dass** mindestens einer (5) der Schenkel (4, 5) an seinem freien Ende (5A) breiter wird.

5. Prothese nach einem der Ansprüche 1 bis 4, **dadurch gekennzeichnet, dass** die Umhüllung (6) im Bereich des freien Endes (4A, 5A) mindestens eines der Schenkel (4, 5) eine Umstülpung (16) aufweist.

6. Prothese nach einem der vorhergehenden Ansprüche, **dadurch gekennzeichnet, dass** die Umhüllung (6) mindestens an ihrem Ende, das dem freien Ende (4A, 5A) der Schenkel (4, 5) entgegengesetzt ist, genäht ist.

7. Prothese nach einem der vorhergehenden Ansprüche, **dadurch gekennzeichnet, dass** sie mindestens ein röntgendichtes Element (6B, 15) umfasst.

8. Prothese nach Anspruch 7, **dadurch gekennzeichnet, dass** sie mindestens ein röntgendichtes Element (15) umfasst, das am freien Ende (4A, 5A) mindestens eines der Schenkel (4, 5) angeordnet ist.

9. Prothese nach einem der Ansprüche 7 und 8, **dadurch gekennzeichnet, dass** sie mindestens zwei röntgendichte Elemente (15) umfasst, die an der Struktur (2) derart angeordnet sind, dass sie in der implantierten Position die Abzweigung mindestens einer (A1) der Nierenarterien (A1, A2) abgrenzen.

10. Prothese nach einem der Ansprüche 7 bis 9, **dadurch gekennzeichnet, dass** sie eine Vielzahl von röntgendichten Elementen (6B, 15) umfasst, die V-förmig angeordnet sind und den Verzweigungspunkt zwischen den beiden Schenkeln (4, 5) jeweils an den beiden Seiten des Stammes (3) mit dem Ende der Umhüllung (6) verbinden, das den Schenkeln (4, 5) entgegengesetzt ist.

11. Prothese nach einem der vorhergehenden Ansprüche, **dadurch gekennzeichnet, dass** die Struktur (2) einen Endteil (3A) aufweist, der nicht von der Umhüllung (6) bedeckt ist und zur Befestigung der Struktur (2) an der Aorta (AO) im Bereich der Nierenarterien (A1, A2) bestimmt ist.

12. Prothese nach einem der vorhergehenden Ansprüche, **dadurch gekennzeichnet, dass** die Struktur (2) mindestens teilweise in Form eines Geflechts (13) gebildet ist und mindestens eine wellige Faser (F) umfasst, die im Wesentlichen ringförmige, untereinander verbundene Einheiten (UA) bildet, dass mindestens manche der Wellen (ON) der welligen Faser (F) von jeweils zwei aneinandergrenzenden Einheiten (UA) durch Verbindungsmittel (14) zwei zu zwei verbunden sind, dass mindestens manche der Verbindungsmittel (14) Glieder (6A, 6B) umfassen, die aus einem starren Teil gebildet sind und die mit mindestens zwei Schleifen (B1, B2) versehen sind, die fest miteinander verbunden sind, und dass bei jedem der Glieder (6A, 6B) jede der beiden Schleifen (B1, B2) des Gliedes mit Spiel (J) jeweils die beiden zu verbindenden Wellen (ON) einschließt.

13. Prothese nach Anspruch 12, **dadurch gekennzeichnet, dass** mindestens eines (6B) der Glieder (6A, 6B) röntgendicht ist.
